# EUROPEAN PATENT APPLICATION

(11) **EP 1 637 162 A1**
(43) Date of publication of application: **22.03.2006**
(21) Application number: 04745799.9
(22) Date of filing: 11.06.2004
(51) Int. Cl.: A61K 45/00, A61K 38/22, A61P 37/00, A61P 1/02, A61P 1/04, A61P 3/10, A61P 7/06, A61P 9/00, A61P 17/00, A61P 17/06, A61P 19/02, A61P 21/00, A61P 21/04, A61P 25/00, A61P 29/00, A61P 43/00

(54) **MEDICINAL COMPOSITION FOR PREVENTING OR TREATING Th1 TYPE IMMUNOLOGICAL DISEASE**

(30) Priority: 13.06.2003 JP 2003169370
(71) Applicant: Daiichi Suntory Pharma Co., Ltd., Tokyo 102-8530 (JP)
(72) Inventor: HORI, Toshiyuki, Kyoto-shi, Kyoto 6060841 (JP)
(74) Representative: HOFFMANN EITLE
(86) International application number: PCT/JP2004/008205
(87) International publication number: WO 2004/110489

(57) **Abstract**

[Problems] The present invention provides a pharmaceutical composition for preventing or treating Th1-mediated immune diseases.

[Means for Solving] There is obtained a pharmaceutical composition provided for prevention or treatment of Th1-mediated immune diseases, which comprises as an active ingredient a substance capable of acting on the NP receptor GC-A expressed on dendritic cells to enhance cGMP production and thereby driving T cells to differentiate into Th2-type cells by regulating cytokine production from dendritic cells.

## Description

### TECHNICAL FIELD

The present invention relates to a pharmaceutical composition provided for the prevention or treatment of Th1-mediated immune diseases, which comprises as an active ingredient a substance capable of acting on the natriuretic peptide (NP) receptor guanylyl cyclase A (GC-A) expressed on dendritic cells to enhance the production of cyclic guanosine monophosphate (cGMP).

### BACKGROUND ART

The immune system has been originally evolved as a defense mechanism for recognizing and eliminating foreign bodies (e.g., microorganisms). To this end, organisms distinguish their own cells or tissues from foreign bodies (non-self antigens) and have developed acquired immunity to eliminate non-self antigens rapidly and efficiently such that they do neither respond to self-antigens nor mount immune responses (immunological tolerance) even if they respond to self-antigens. In the immune system, T cells play a predominant role. Undifferentiated naive T cells in the periphery (Thp) start to proliferate and differentiate upon antigen stimulation. In this case, Thp cells are activated through T cell receptors on their surface in response to both antigen presentation by antigen-presenting cells (e.g., macrophages or dendritic cells) and signals from activation-related molecules, so that the Thp cells secrete IL-2 and proliferate. After that, the activated Thp cells differentiate into Th0 cells capable of producing almost all cytokines and functionally mature into Th1 or Th2 cells in accordance with the final direction of their differentiation, which is determined depending on, for example, the type and strength of antigen stimulation or stimulation signals from antigen-presenting cells, thereby inducing the production of cytokines specific to each cell type, further proliferation, cytotoxic activity, and so on. Namely, interleukin-12 (IL-12) induces differentiation into Th1 cells involved in cellular immunity, and the differentiated Th1 cells produce cytokines such as interleukin-2 (IL-2) and interferon-γ (IFN-γ). In contrast, interleukin-4 (IL-4) induces differentiation into Th2 cells involved in humoral immunity, which produce cytokines such as interleukins-4 and 10 (IL-4 and 10). Cytokines produced by each of the cell types mutually and negatively regulate differentiation into Th1 or Th2 cells and the action of cytokines produced by each of the cell types to maintain a proper Th1/Th2 balance.

In recent years, it has come to be believed that an imbalance of this Th1/Th2 balance may be responsible for the onset of immune diseases. When an immune response is biased toward Th1-dominant immunity (Th1-mediated immunity), cellular immunity is enhanced to stimulate immune reactions against cancers and infections, but self-tissue damage is also caused and hence responsible for the onset of autoimmune diseases. Tissue damage and infection further cause inflammatory reactions, which in turn lead to tissue fibrosis and/or organ dysfunction.

Even in the case of normal immune responses, it is also therapeutically desired to inhibit graft rejection following organ transplantation and/or graft-versus-host disease caused by bone marrow (hematopoietic stem cell) transplantation. Immune responses induced by these events are identical in principle and are predominantly based on Th1-mediated immunity.

Examples of Th1-mediated immune diseases caused by abnormal immunity due to Th1-mediated immunity include graft rejection following transplantation, graft-versus-host disease caused by bone marrow (hematopoietic stem cell) transplantation, and autoimmune diseases such as autoimmune hepatitis, chronic rheumatoid arthritis, insulin-dependent diabetes mellitus, ulcerative colitis, Crohn's disease, multiple sclerosis, autoimmune myocarditis, psoriasis, scleroderma, myasthenia gravis, multiple myositis/dermatomyositis, Hashimoto's disease, autoimmune hypocytosis (e.g., pure red cell aplasia, aplastic anemia), Sjogren's syndrome, vasculitis syndrome, and systemic lupus erythematosus, as well as tissue damage caused by these diseases, inflammatory reactions associated with infection, fibrosis, organ dysfunction, etc.

To treat autoimmune diseases and/or transplantation immunity due to Th1-mediated immunity, as well as chronic active immunological inflammatory reactions associated with these diseases, selective inhibition of Th1-mediated immunity has been desired. Under these circumstances, current expectations are placed on therapies involving mediator control in the T cell activation mechanism, and attention is given to strong immunosuppressive effects of cyclosporin and FK506 against T cells, anti-cytokine therapy, anti-adhesion molecule (activation-related molecule) therapy, monoclonal antibody therapy, etc.

However, these therapies cannot be regarded as being selective to Th1-mediated immunity, as in the case of conventional therapies using steroids, or certain types of immunosuppressive agents which act on nucleic acid synthesis systems, or interferon formulations. There still remain unsolved problems, such as various side effects including infectious exacerbation, diabetes, thrombosis, moon face, nephropathy and fervescence, and therefore the development of pharmaceutical agents with improved safety and efficacy has been desired.

Autoimmune diseases are systemic and also observed in almost every organ. In the future, further advances in organ transplantation surgery and hematopoietic stem cell transplantation will increase the problem of graft rejection or graft-versus-host disease. In view of the foregoing, diseases in which Th1-mediated immunity plays a predominant role include a wide range of diseases, and there is a demand for the development of effective therapeutic agents for these diseases. Agents reported to have a suppressive effect on Th1-mediated immunity include serotonin 1A receptor antagonists (J Immunol 153:489-498, 1994), pentoxifyllin (J Cardiovasc Pharmacol 25 Suppl 2:S75-79, 1995), beta 2-adrenergic receptor agonists (J Immunol 158:4200-4210, 1997, J Clin Invest 100:1513-1519, 1997), iloprost as an adenylate cyclase activator (J Autoimmun 10:519-529, 1997), pyridinyl imidazole compounds and SB203580 as P38 MAP kinase inhibitors (EMBO J 17:2817-2829, 1998, Int Immunol 12:253-261, 2000), lisofylline (J Immunol 163:6567-6574, 1999), CGS-21680 as an adenosine A2a receptor agonist (J Immunol 164:436-442, 2000), NO-aspirin (Gastroenterology 118:404-421, 2000) and 1,25-dihydroxyvitamin D(3) (Eur J Immunol 30:498-508, 2000). However, no clinically applicable pharmaceutical preparation has been developed that enables selective inhibition of Th1-mediated immunity and reduction of side effects.

Recent studies have elucidated that Crohn's disease is a Th1-mediated disease, and attention is given to IL-12 and IL-10 as therapeutic targets for this disease (Saishin Igaku (the Latest Medicine) 90:1076-1081, 2004). Namely, IL-12 is secreted from activated macrophages or dendritic cells and plays a predominant role in the differentiation of naive T cells into Th1 cells. An anti-IL-12 antibody is reported to show a therapeutic effect in animal models and IL-12 is therefore believed to be a potential therapeutic target for human Crohn's disease. Likewise, IL-10 is a molecule inhibiting cytokine production from Th1 cells. Since IL-10 knockout mice show an enhanced Th1 response and will spontaneously develop enteritis (Clin Invest Med 2001; 24: 250-257), IL-10 is also expected as a therapeutic target molecule for Crohn's disease. Recombinant human IL-10 was actually studied for its effect on Crohn's disease and found to produce an ameliorating effect, but its efficacy was not sufficient and side effects such as headache, fervescence and anaemia were also observed (Gastroenterology, 2000; 119: 1473-1482). It has been suggested that IL-10 produces an immune-activating effect when maintained at high concentrations in the blood, and there has been a demand for the development of a therapy that enables IL-10 production with the physiological levels only at the diseased site. Recently, the anti-TNFα antibody infliximab has been applied for treatment of moderate to severe Crohn's disease and confirmed to have efficacy. However, this antibody is reported to have a high frequency of side effects, and its use is therefore limited.

On the other hand, multiple sclerosis is a disease in which Th1-mediated immunity is particularly dominant among autoimmune diseases. Even in the relapsing-remission type, the effect of driving Th2 polaraization is expected to lead to treatment of pathological conditions because Th2 polarization is observed during remission. Currently used therapeutic agents for multiple sclerosis include steroids for the acute stage, interferon β1b for the relapsing-remission type, and various immunosuppressive agents for the primary progressive type. Since these agents are pointed out to have various side effects and their efficacy is also insufficient, there still remains a need to ensure reliable improvement of symptoms, prevention of disease progression, and reduction of side effects.

For these two diseases, it is therefore desired to develop a pharmaceutical preparation that enables more selective inhibition of Th1-mediated immunity and reduction of side effects, but there is no pharmaceutical preparation clinically applicable at present for this purpose.

Dendritic cells are the only antigen-presenting cells having the strongest ability to activate naive T cells in the T regions of lymphoid organs and are also known to play an important role in maintaining the homeostasis of self-defense system (Banchereau, J. et al. Nature, Vol. 392, p. 245, 1998). Although dendritic cells are generally present in tissues in an immature state with low ability to activate T cells, they will cause binding antigen-derived peptides with MHC molecules and will present the peptide-MHC complexes to naive T cells when stimulated for maturation and activation by pathogens and/or inflammatory mediators released from damaged tissues. At the same time, dendritic cells enhance the expression of co-stimulation molecules, cause the production of various cytokines, drive the recruitment of T cells, and activate antigen-specific cells to induce immune responses (Iyoda Tomonori, Inaba Kayo, Protein, Nucleic Acid and Enzyme, Vol. 47, p. 2133, 2002). Moreover, human dendritic cells have at least two types of progenitor cells. Progenitor cells of monocyte lineage differentiate upon GM-CSF and IL-4 stimulation and further drive naive T cells to differentiate into Th1 cells by the action of IL-12 produced upon CD40L stimulation. Progenitor cells of plasma cell lineage differentiate into dendritic cells having little ability to produce IL-12 upon stimulation by viruses and/or CpG oligonucleotides as well as IL-3 and CD40L, and drive naive T cells to differentiate into Th2 cells (Ohteki Toshiaki, Igaku no Ayumi (Progress in Medicine), Vol. 205, p. 57, 2003). Namely, dendritic cells play an important role in regulating the Th1/Th2 balance in the immune system. Thus, if a pharmaceutical agent can be developed that specifically acts on dendritic cells to regulate their activity or cytokine expression and hence drives naive T cells toward Th2 deviation by inhibiting their differentiation and proliferation into Th1 cells, such a pharmaceutical agent can be expected to act as a more fundamental therapeutic or prophylactic agent for abnormal immunity (particularly Crohn's disease or multiple sclerosis) due to Th1-mediated immunity. However, until now, there has been no such promising pharmaceutical agent available that has such an effect.

On the other hand, peptidic substances, particularly natriuretic peptides, can be exemplified as substances capable of acting on GC-A to enhance the production of cGMP, which is a second messenger. Three types of natriuretic peptides (NPs) have been known: ANP (atrial natriuretic peptide), BNP (brain natriuretic peptide) and CNP (C-type natriuretic peptide), and three types of NP receptors for these peptides have been identified: GC-A, GC-B (guanylyl cyclase A, B) and NPR-C (NP receptor-C). It has been found that GC-A and GC-B have a membrane-bound guanylyl cyclase structure, that ANP and BNP are specific ligands for GC-A, while CNP is a specific ligand for GC-B, and that these peptides increase intracellular cGMP levels to thereby produce physiological actions such as diuretic action and vasodilator action after binding to their respective receptors. It is also considered that NPR-C is not coupled with cGMP production, and is involved in the metabolism and/or clearance of these hormones (Suzuki, T. et al. Cardiovasc. Res. Vol. 51, p. 489, 2001).
ANP is a peptide hormone that is secreted from the heart and plays an important role in water-electrolyte metabolism and blood pressure regulation. In human subjects and animal models, blood ANP concentrations are known to increase with the severity of cardiac hypertrophy and heart failure; ANP is believed to act on pathological conditions in heart failure in a compensating manner. ANP administration is actually found to induce vasodilator action and diuretic action in heart failure patients, thus reducing both preload and afterload on the heart and resulting in an improvement in hemodynamics (Suzuki, T. et al. Cardiovasc. Res. Vol. 51, p. 489, 2001).

The ANP receptor GC-A is expressed not only in the cardiovascular system, but also in leukocytes, suggesting a possibility that ANP may have physiological functions on hemocytic cells. More specifically, ANP is reported to promote neutrophil migration (Izumi, T. et al. J. Clin. Invest. Vol. 108, p. 203, 2001), to inhibit the proliferation of rat thymocytes (Vollmar, A. M., K. N., et al. Endocrinology. Vol. 137, 1706, 1996), to enhance the cytotoxicity of human natural killer (NK) cells (Moss, R. B., and M. G. Golightly, Peptides, Vol. 2, p. 851, 1991) and to inhibit the production of NO or TNFα from mouse macrophages (Kiemer, A. K., and A. M. Vollmar. J. Biol. Chem. Vol. 273, p. 13444, 1998), etc.

However, unlike mouse monocyte-derived macrophages, human monocytes do not express ANP receptors and ANP is reported to have no physiological activity (including cGMP production) on human monocytes (Sprenger H., et al., Immunobiol. Vol. 183, p. 94, 1991). Thus, with respect to substances capable of acting on GC-A to enhance cGMP production, there has been no report on their physiological functions, pathophysiological significance or immunomodulatory effects in human monocyte-derived dendritic cells involved in the immune system.

### DISCLOSURE OF THE INVENTION

### PROBLEMS TO BE SOLVED BY THE INVENTION

Under these circumstances, the object of the present invention is to provide a side-effect-free and clinically-applicable inhibitor selective to Th1-mediated immunity, the mechanism of which relies on the inhibition of Th1-mediated cytokine production and Th1 cell proliferation/function and which is targeted for Th1-mediated immune diseases including autoimmune diseases, graft rejection following organ transplantation, graft-versus-host disease caused by bone marrow (hematopoietic stem cell) transplantation, tissue damage due to autoimmune diseases and relative diseases, inflammatory reactions associated with infection, as well as tissue fibrosis and organ dysfunction. More specifically, the object of the present invention is to provide a pharmaceutical composition for preventing or treating Th1-mediated immune diseases (particularly Crohn's disease or multiple sclerosis), which comprises as an active ingredient a substance capable of acting on the NP receptor GC-A expressed on dendritic cells to enhance cGMP production, thereby regulating cytokine production in the dendritic cells and driving T cell differentiation into Th2 cells.

### MEANS FOR SOLVING THE PROBLEMS

Any substance may be used as an active ingredient in the pharmaceutical composition of the present invention, as long as it has the property of enhancing cGMP production through the NP receptor GC-A. Preferred are peptidic substances, but it is also possible to use any compound other than peptidic substances as long as it is capable of acting on the NP receptor GC-A to enhance cGMP because there is no particular limitation on the active ingredient.

Preferred peptidic substances are natriuretic peptides including atrial natriuretic peptide (hereinafter referred to as ANP), brain natriuretic peptide (hereinafter referred to as BNP) and the like.

Although human-derived α-hANP of 28 amino acids (SEQ ID NO: 1) or rat-derived α-rANP of 28 amino acids (SEQ ID NO: 2) can be used as ANP, the peptide used as an active ingredient in the present invention may be a peptide having the ring structure of ANP (Cys-based disulfide linking) and the C-terminal region following the ring structure. Examples of such a peptide include a peptide covering amino acid residues at positions 7-28 of α-hANP (SEQ ID NO: 3). A particularly desired ANP is human-derived α-hANP.

Examples of BNP include human BNP of 32 amino acids (SEQ ID NO: 4) and the like.

Moreover, the substance of the present invention that has the property of enhancing cGMP production through the NP receptor GC-A may be isolated in pure form from natural sources, chemically synthesized or recombinantly produced. For example, based on the amino acid sequence of the above substance (e.g., α-hANP), those skilled in the art can obtain an appropriate substance in a known manner by modification such as deletion, substitution, addition and/or insertion of amino acid residues in the sequence. Any substance obtained in any of these manners can be used as long as it is a substance capable of acting on the NP receptor GC-A to enhance cGMP production. Examples of such a substance include, in addition to those listed above, frog ANP (SEQ ID NO: 5), pig BNP (SEQ ID NO: 6), rat BNP (SEQ ID NO: 7), chick NP (SEQ ID NO: 8) and the like.

The substance available for use as an active ingredient in the pharmaceutical composition of the present invention may be used in the form of an acid addition salt with an inorganic acid (e.g., hydrochloric acid, sulfuric acid, phosphoric acid) or with an organic acid (e.g., formic acid, acetic acid, butyric acid, succinic acid, citric acid). The substance may also be in the form of a metal salt (e.g., sodium, potassium, lithium or calcium salt) or in the form of a salt with an organic base. Alternatively, in the pharmaceutical composition of the present invention, the substance used as an active ingredient may be in free form or in pharmaceutically acceptable salt form.

The substance or its pharmacologically acceptable salt available for use as an active ingredient in the pharmaceutical composition of the present invention is preferably administered in admixture with a known pharmacologically acceptable carrier, excipient, diluent or the like in any manner commonly used for administration of pharmaceutical preparations, i.e., by the oral route or by the parenteral route (e.g., intravenous, intramuscular or subcutaneous route).

In a case where the active ingredient is a peptidic substance, it may also be formulated into a formulation resistant to decomposition in the digestive tract, for example, microcapsules encapsulating the active ingredient peptide within liposomes, and administered by the oral route. Alternatively, it is possible to use any transmucosal route (e.g., rectal, intranasal or sublingual route), in addition to absorption from the digestive tract. In this case, the active ingredient can be administered in the form of suppositories, nasal sprays, sublingual tablets, and so on.

The dose of the substance available for use as an active ingredient in the pharmaceutical composition of the present invention will vary depending on the type of disease, the age and body weight of a patient, the severity of symptoms, the route of administration, etc. In general, the substance may be administered over a range of 0.1 µg/kg to 100 mg/kg, preferably 0.5 µg/kg to 5 mg/kg.

The present invention has demonstrated that a composition comprising as an active ingredient a substance capable of acting on the natriuretic peptide receptor GC-A to enhance cGMP production is effective against Th1-mediated immune diseases because it specifically acts on dendritic cells to regulate their cytokine production and thereby drives naive T cells toward Th2 polarization to inhibit Th1-mediated immune reactions. In particular, when ANP was used as an active ingredient, ANP alone had no effect on cytokine production from dendritic cells or proliferation of T cells and showed a regulatory effect only on LPS (lipopolysaccharide)-stimulated responses. This means that ANP does not greatly affect normal immune functions and inhibits only overresponses upon stimulation, i.e., that ANP has fewer side effects and can be used safely. ANP is therefore useful.

Incidentally, LPS is a major component of the external membrane of Gram-negative bacteria and is very important for recognition of components inherent to pathogens. When recognized by TLR4 among the group of membrane protein receptors called the Toll-like receptor (TLR) family expressed on dendritic cells, LPS stimulates maturation and activation of the dendritic cells to induce the expression of cytokines and functional accessory molecules such as CD40.

According to the present invention, a series of experiments using human-derived dendritic cells and naive T cells have shown that GC-A is expressed on human dendritic cells, and that the substance capable of acting on the NP receptor GC-A to enhance cGMP production has a regulatory effect on cytokine production in the dendritic cells and also has the effect of polarizing T cells toward Th2-promoting phenotype. These results are clinically useful.

In view of the foregoing, it is indicated that the substance capable of acting on the NP receptor GC-A to enhance cGMP production has the effect of acting on GC-A expressed on dendritic cells to polarize naive T cells toward Th2-promoting phenotype and hence can regulate the Th1/Th2 balance of T cells in the immune system. The substance may therefore be administered to ameliorate Th1-mediated immune diseases (particularly Crohn's disease or multiple sclerosis). With respect to the pre-disease state in which Th1-mediated immune diseases have not appeared, if the proportion of Th1 is higher than that of Th2 as measured in a standard manner, the substance may also be administered to regulate the Th1/Th2 balance in the immune system, thereby preventing the onset of Th1-mediated immune diseases.

In view of the foregoing, the present invention encompasses the following aspects.
(1) A pharmaceutical composition for preventing or treating a Th1-mediated immune disease, which comprises as an active ingredient a substance capable of acting on the natriuretic peptide receptor guanylyl cyclase A to enhance the production of cyclic guanosine monophosphate.
(2) The pharmaceutical composition according to (1) above, wherein the Th1-mediated immune disease is selected from a disease due to graft rejection following transplantation, graft-versus-host disease caused by bone marrow (hematopoietic stem cell) transplantation, and an autoimmune disease.
(3) The pharmaceutical composition according to (2) above, wherein the autoimmune disease is selected from autoimmune hepatitis, chronic rheumatoid arthritis, insulin-dependent diabetes mellitus, ulcerative colitis, Crohn's disease, multiple sclerosis, autoimmune myocarditis, psoriasis, scleroderma, myasthenia gravis, multiple myositis/dermatomyositis, Hashimoto's disease, autoimmune hypocytosis (e.g., pure red cell aplasia, aplastic anemia), Sjogren's syndrome, vasculitis syndrome, and systemic lupus erythematosus.
(4) The pharmaceutical composition according to (3) above, wherein the autoimmune disease is Crohn's disease or multiple sclerosis.
(5) A pharmaceutical composition for preventing or treating tissue damage due to a Th1-mediated immune disease, an inflammatory reaction associated with infection, fibrosis or organ dysfunction, which comprises as an active ingredient a substance capable of acting on the natriuretic peptide receptor guanylyl cyclase A to enhance the production of cyclic guanosine monophosphate.
(6) The pharmaceutical composition according to (1) or
(5) above, wherein the substance capable of acting on the natriuretic peptide receptor guanylyl cyclase A to enhance the production of cyclic guanosine monophosphate is a natriuretic peptide.
(7) The pharmaceutical composition according to (6) above, wherein the natriuretic peptide is atrial natriuretic peptide or brain natriuretic peptide.
(8) The pharmaceutical composition according to (7) above, wherein the atrial natriuretic peptide is of human origin.
(9) A method for treating a Th1-mediated immune disease, which comprises administering a substance capable of acting on the natriuretic peptide receptor guanylyl cyclase A to enhance the production of cyclic guanosine monophosphate.
(10) The method according to (9) above, wherein the Th1-mediated immune disease is selected from a disease due to graft rejection following transplantation, graft-versus-host disease caused by bone marrow (hematopoietic stem cell) transplantation, and an autoimmune disease.
(11) The method according to (10) above, wherein the autoimmune disease is selected from autoimmune hepatitis, chronic rheumatoid arthritis, insulin-dependent diabetes mellitus, ulcerative colitis, Crohn's disease, multiple sclerosis, autoimmune myocarditis, psoriasis, scleroderma, myasthenia gravis, multiple myositis/dermatomyositis, Hashimoto's disease, autoimmune hypocytosis (e.g., pure red cell aplasia, aplastic anemia), Sjogren's syndrome, vasculitis syndrome, and systemic lupus erythematosus.
(12) The method according to (11) above, wherein the autoimmune disease is Crohn's disease or multiple sclerosis.
(13) The method according to (9) above, wherein the substance capable of acting on the natriuretic peptide receptor guanylyl cyclase A to enhance the production of cyclic guanosine monophosphate is a natriuretic peptide.
(14) The method according to (13) above, wherein the natriuretic peptide is atrial natriuretic peptide or brain natriuretic peptide.
(15) The method according to (14) above, wherein the atrial natriuretic peptide is of human origin.
(16) Use of a substance capable of acting on the natriuretic peptide receptor guanylyl cyclase A to enhance the production of cyclic guanosine monophosphate for the manufacture of a pharmaceutical composition for preventing or treating a Th1-mediated immune disease.
(17) The use according to (16) above, wherein the Th1-mediated immune disease is selected from a disease due to graft rejection following transplantation, graft-versus-host disease caused by bone marrow (hematopoietic stem cell) transplantation, and an autoimmune disease.
(18) The use according to (17) above, wherein the autoimmune disease is selected from autoimmune hepatitis, chronic rheumatoid arthritis, insulin-dependent diabetes mellitus, ulcerative colitis, Crohn's disease, multiple sclerosis, autoimmune myocarditis, psoriasis, scleroderma, myasthenia gravis, multiple myositis/dermatomyositis, Hashimoto's disease, autoimmune hypocytosis (e.g., pure red cell aplasia, aplastic anemia), Sjogren's syndrome, vasculitis syndrome, and systemic lupus erythematosus.
(19) The use according to (18) above, wherein the autoimmune disease is Crohn's disease or multiple sclerosis.
(20) The use according to (16) above, wherein the substance capable of acting on the natriuretic peptide receptor guanylyl cyclase A to enhance the production of cyclic guanosine monophosphate is a natriuretic peptide.
(21) The use according to (20) above, wherein the natriuretic peptide is atrial natriuretic peptide or brain natriuretic peptide.
(22) The use according to (21) above, wherein the atrial natriuretic peptide is of human origin.
(23) A method for regulating the Th1/Th2 balance in the immune system, which comprises treating dendritic cells with a substance capable of acting on the natriuretic peptide receptor guanylyl cyclase A to enhance the production of cyclic guanosine monophosphate, and thereby polarizing T cells toward Th2-promoting phenotype.
(24) The method according to (23) above, wherein the substance capable of acting on the natriuretic peptide receptor guanylyl cyclase A to enhance the production of cyclic guanosine monophosphate is a natriuretic peptide.
(25) The method according to (24) above, wherein the natriuretic peptide is atrial natriuretic peptide or brain natriuretic peptide.
(26) The method according to (25) above, wherein the atrial natriuretic peptide is of human origin.

### EFFECTS OF THE INVENTION

According to the present invention, substances capable of acting on the natriuretic peptide receptor guanylyl cyclase A to enhance the production of cyclic guanosine monophosphate have the effect of inhibiting Th1-mediated immune reactions because they act on dendritic cells to induce Th2 polarization and hence drive T cells to differentiate into Th2-type cells, thereby inhibiting IL-12 and TNFα production and enhancing IL-10 production. Thus, pharmaceutical compositions comprising such a substance as an active ingredient are very useful as pharmaceutical compositions for preventing or treating Th1-mediated immune diseases (particularly Crohn's disease or multiple sclerosis) by regulating the Th1/Th2 balance in the immune system.

### BRIEF DESCRIPTION OF DRAWINGS

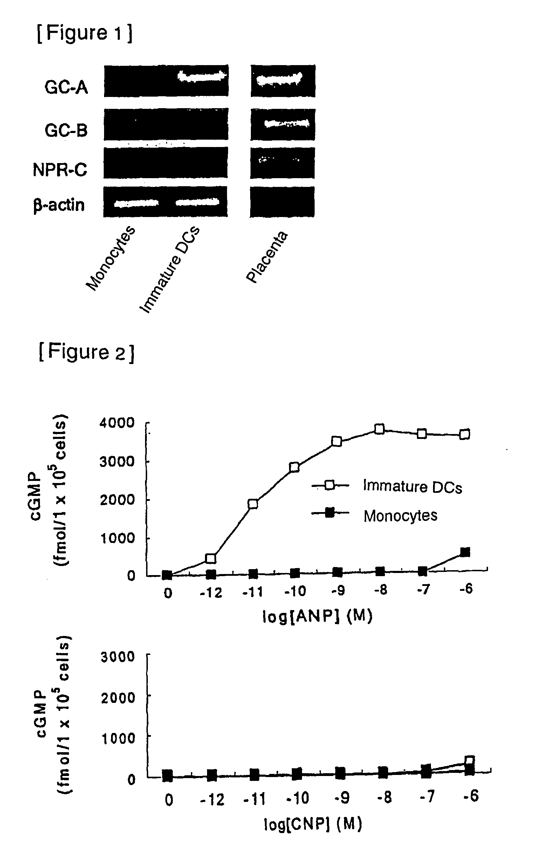

Figure 1 shows the RT-PCR results of mRNA expression analyzed for three NP receptors (i.e., GC-A, GC-B and NPR-C) in human monocytes (monocytes) and immature dendritic cells (immature DCs). The placenta was used as a positive control for each receptor mRNA. Moreover, RNA integrity and cDNA synthesis were verified by amplification of β-actin cDNA. This figure indicates that GC-A mRNA is specifically expressed only in immature dendritic cells.
Figure 2 shows the cGMP producing activity of ANP (upper panel) and CNP (lower panel) in human monocytes (monocytes, solid square) and immature dendritic cells (immature DCs, open square). Each value is expressed as the cGMP level per 1 × 10⁵ cells. This figure indicates that ANP enhances cGMP production in dendritic cells even at extremely low concentrations.

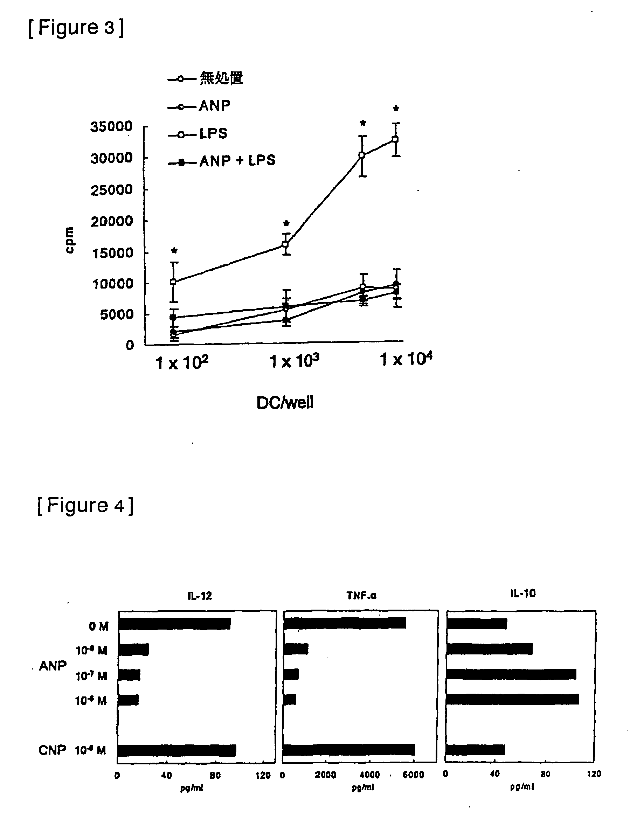

Figure 3 shows effects of ANP on the proliferative response of allogeneic naive CD4 T cells to LPS-stimulated DCs. Dendritic cells were cultured for 24 hours in the presence or absence of LPS (1 µg/mL), ANP (10⁻⁷M) or LPS+ANP and, after irradiation, were then cultured together with naive T cells for an additional 6 days. The cell proliferation ability of naive T cells at this time was evaluated by [³H]-thymidine uptake. Symbols: open circle, untreated cells; solid circle, cells treated with ANP (10⁻⁷ M); open square, cells treated with LPS (1 µg/mL); and solid square, cells treated with ANP+LPS. Each value is expressed as mean ± standard error of five experiments. * denotes a statistically significant difference at p<0.05 in comparison with the other groups. Statistically significant differences were determined by using Student's t-test.
Figure 4 shows the influence of ANP and CNP on cytokine production from LPS-stimulated dendritic cells, expressed as the immunoreactivity of IL-12, TNF-α or IL-10 in medium after 24 hour incubation of dendritic cells (1 × 10⁵ cells/tube) together with LPS (1 µg/mL) in the presence or absence of ANP (10⁻⁸ to 10⁻⁶ M) or CNP (10⁻⁶ M).

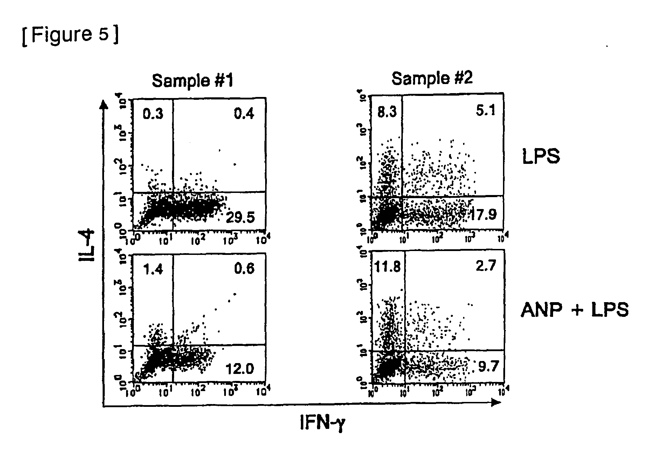

Figure 5 shows the results of flow cytometric analysis for intracellular IFN-γ or IL-4 production in naive T cells which were cultured together with LPS- or LPS+ANP-pretreated dendritic cells and further grown in an IL-2-containing culture medium. Numbers in the figure represent the percentage of leukocytes in the individual fractions. Sample #1 and Sample #2 show the results obtained with dendritic cells derived from different subjects.

### BEST MODE FOR CARRYING OUT THE INVENTION

The inventors of the present invention studied ANP receptor (GC-A) expression in dendritic cells isolated and cultured from human peripheral blood and the cGMP production-enhancing activity of ANP, and also studied the effect of ANP on dendritic cell differentiation, lymphocyte proliferation, cytokine expression and Th1/Th2 deviation to elucidate physiological functions of ANP in dendritic cells. As a result, the inventors found that a substance capable of acting on GC-A to enhance cGMP production was useful for prevention or treatment of Th1-mediated immune diseases.

### A. Isolation of human dendritic cells and experimental procedures for NP receptor expression and stimulation of cGMP production

1. Isolation and culturing of human peripheral blood-derived dendritic cells
   For experiments, the leukocyte layer (buffy coats) of peripheral blood from healthy volunteers (provided by Kyoto Red Cross Blood Center, Japan) was used. After the peripheral blood monocyte fraction was separated by density gradient centrifugation using Ficoll-Paque, the fraction was applied to a magnetic bead column using MACS CD14 or cultured in cell culture flasks at 37°C for 1 hour to select the adhered cells, thereby separating monocytes. Immature dendritic cells were obtained by culturing the monocytes (2 × 10⁵ cells/ml) for 7 days at 37°C in the presence of 10% fetal bovine serum, 50 ng/mL human GM-CSF and 20 ng/mL human IL-4.

2. RT-PCR of NP receptors
After total RNA was extracted from the monocytes or the dendritic cells using an RNA isolation kit, 1 µg of total RNA and oligo(dT) primers were used to synthesize single-stranded cDNA by avian myeloblastosis virus-derived reverse transcriptase. The primers used are shown below.

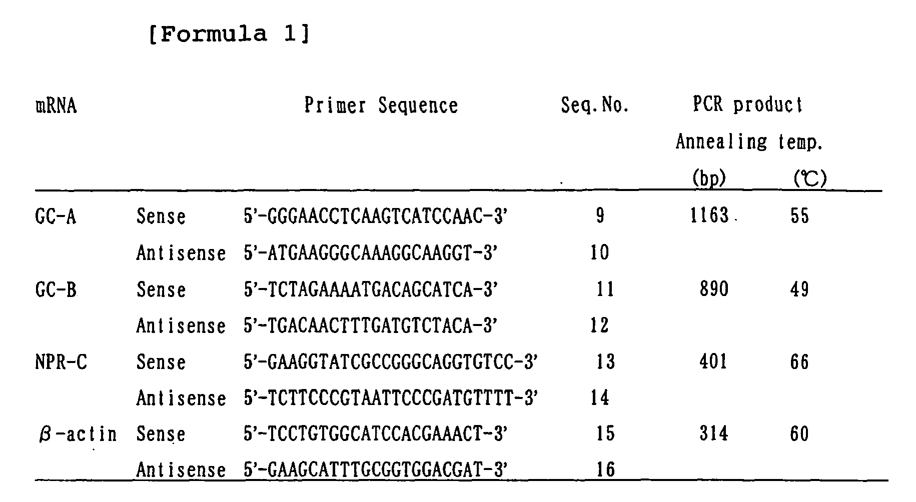

PCR was repeated 35 cycles for NP receptors and 25 cycles for β-actin. The PCR products were separated on a 1.5% to 2% agarose gel, stained with ethidium bromide and then detected with a UV transilluminator.

3. Measurement of cGMP producing activity
After the cells (1 × 10⁵ cells/sample) were incubated in 500 µL of 10 mM HEPES, 0.5 mM 3-isobutyl-1-methylxanthine and 1 µM phospholamidon at 37°C for 10 minutes, ANP or CNP was added to a final concentration of 10⁻¹² to 10⁻⁶ M and incubation was continued for an additional 15 minutes. After the cells were washed and lysed, the intracellular cGMP concentration was measured by the ELISA method.

### B. Experimental procedures for dendritic cell-induced naive T cell proliferation, cytokine production and naive T cell differentiation

1. T cell proliferation induced by activated dendritic cells
   Naive T cells (naive CD4⁺ T cells) were isolated from the monocyte fraction of umbilical cord blood using magnetic beads for MACS CD4⁺ T cell isolation. Dendritic cells were activated by being cultured for 24 hours with lipopolysaccharide (LPS, 1 µg/mL) in the presence or absence of ANP (10⁻⁷ M). The cells were washed and irradiated (30 Gy) to eliminate their proliferation ability, followed by culturing together with the naive T cells (1 × 10⁵ cells/well) for 6 days. The T cells were evaluated for their proliferation ability by 8 hour uptake of [methyl-³H]-thymidine (0.5 µCi/well).

2. Measurement of cytokine production from dendritic cells
Dendritic cells were incubated for 24 hours with LPS (1 µg/mL) in the presence or absence of ANP (10⁻⁸ M to 10⁻⁶ M) or CNP (10⁻⁶ M), and the levels of IL-12, IL-10 and TNFα in each culture supernatant were measured by the ELISA method.

3. Intracellular cytokine expression analysis on naive T cells (to study dendritic cell-driven Th1/Th2 polarization)
Dendritic cells were activated by being pre-incubated for 24 hours with LPS (1 µg/mL) in the presence or absence of ANP (10⁻⁷ M) and then irradiated (30 Gy) to eliminate their proliferation ability. These dendritic cells (1 × 10⁵ cells/well) and naive T cells separated from cord blood (1 × 10⁶ cells/well) were co-cultured for 6 days. After the T cells were grown in the presence of IL-2 (50 U/mL) for an additional 8 days, the T cells were collected and stimulated for 4 hours with 50 ng/mL phorbol ester (PMA) and 500 ng/mL ionomycin. Two hours before completion of the incubation, Brefeldin A (10 µg/mL) was added. The cells were fixed with 2% formalin and then treated with a medium containing 2% FBS and 0.5% saponin to cause cell membrane damage. Intracellular cytokines were stained with a FITC-labeled anti-IFN-γ monoclonal antibody and a PE-labeled anti-IL-4 monoclonal antibody, followed by flow cytometric analysis. IFN-γ positive cells were determined as Th1-type cells, while IL-4 positive cells were determined as Th2-type cells.

### EXAMPLES

The present invention will be further described in more detail by way of the following examples.
Example 1 GC-A mRNA expression on immature dendritic cells and ANP-induced cGMP production enhancement
The inventors of the present invention first studied whether three NP receptor mRNAs were expressed on dendritic cells or monocytes. RNA was prepared from both monocytes obtained from healthy human peripheral blood and immature dendritic cells, and mRNA expression of the NP receptors GC-A, GC-B and NPR-C was studied by RT-PCR. The results obtained are shown in Figure 1.

The placenta-derived RNA used as a positive control was found to show the expression of all three NP receptor mRNAs. In the monocytes, none of the receptor mRNAs was found to be expressed, whereas in the immature dendritic cells, the expression of only the GC-A mRNA was clearly observed. This confirmed that although the NP receptor mRNAs were not expressed on monocytes, the GC-A mRNA was specifically expressed on monocyte-derived dendritic cells.

Next, to elucidate whether GC-A expressed on dendritic cells was coupled with physiological functions, the cGMP producing activity of ANP was examined in both monocytes and dendritic cells. As a control, CNP was used which was a specific ligand for GC-B. The results obtained are shown in Figure 2.

ANP was found to increase intracellular cGMP levels in dendritic cells in a concentration-dependent manner from a concentration as low as 10⁻¹² M, whereas in monocytes, there was little increase in cGMP levels at all concentrations. Likewise, CNP was found to have no effect on intracellular cGMP levels in either monocytes or dendritic cells. These results support the result of GC-A gene expression shown in Figure 1, and also strongly suggest a possibility that GC-A is expressed in a manner specific to dendritic cells and plays a role in the physiological activity of dendritic cells through binding with ANP.
Example 2 Study of the effect of ANP on dendritic cell cytokine expression and Th2 polarization
The results from Example 1 indicated that ANP was capable of acting on dendritic cells to modulate immune reactions.

An important function of dendritic cells is to prime naive T cells initiating antigen-specific immune reactions. First of all, in the presence or absence of LPS which was known to drive maturation and activation of dendritic cells, ANP was added to dendritic cells and examined for its effect on activation and proliferation of naive T cells. The results obtained are shown in Figure 3.

ANP alone had no effect on proliferation of naive T cells, whereas pronounced T cell proliferation was elicited when LPS was added to the dendritic cells.

On the other hand, when ANP was added simultaneously with LPS, T cell proliferation induced by the LPS-treated dendritic cells was found to be almost completely inhibited.

Thus, ANP was shown to have a potential effect on LPS-induced signals for dendritic cell activation or functions of dendritic cells.

Cytokines produced by dendritic cells are known to play a very important role in developing interactions between dendritic cells and T cells. For this reason, ANP was then examined for its effect on cytokine expression from dendritic cells. The results obtained are shown in Figure 4.

LPS was found to enhance IL-12, TNF-α and IL-10 production from dendritic cells. In contrast, ANP was found to decrease both IL-12 and TNFα levels in a concentration-dependent manner, but found to enhance IL-10 production. It should be noted that LPS-unstimulated dendritic cells had a low ability to produce IL-12, TNF-α and IL-10 and ANP alone had no effect on these cytokine productions.

Moreover, in view of the fact that CNP, a ligand for GC-B, had no effect on cytokine expression, it was confirmed that the effect of ANP observed upon LPS stimulation was mediated by GC-A expressed on dendritic cells.

Among cytokines produced by dendritic cells, IL-12 is a typical Th1-mediated cytokine, while IL-10 is known to block the activity of cytokines (including IL-12) produced by Th1 cells, activated monocytes and NK cells. ANP was found to inhibit IL-12 production from LPS-induced dendritic cells, but found to enhance IL-10 production, indicating a possibility that ANP would polarize dendritic cells toward Th2-promoting phenotype.

To determine whether ANP was able to polarize dendritic cells toward Th2-promoting phenotype, a further experiment was performed to analyze which of Th1 or Th2, naive T cells were differentiated and proliferated when dendritic cells were stimulated with LPS in the presence of ANP. LPS is known to act on the Toll-like receptor 4 in dendritic cells to enhance IL-12 expression, thereby driving naive T cells to deviate to Th1-type cells having a high ability to produce IL-12 (Akira S, et al. Nat. Immunol. Vol. 2, p. 675, 2001).

After the dendritic cells stimulated in the presence of LPS alone or in combination with ANP were allowed to interact with naive T cells, the T cells were grown in a culture solution containing IL-2 and then analyzed by flow cytometory for the levels of IFN-γ (as a cytokine expressed by Th1-type cells) and IL-4 (as a cytokine expressed by Th2-type cells). Figure 5 shows the results analyzed for two T cell specimens. In Figure 5, IFN-γ positive and IL-4 negative cells mean differentiation and proliferation into Th1-type helper T cells, while IL-4 positive and IFN-γ negative cells mean differentiation and proliferation into Th2-type helper T cells.

In both Sample #1 and Sample #2, the groups treated with LPS and ANP were found to clearly show an increase in IL-4-producing cells and a decrease in IFN-γ-producing cells when compared to the groups treated with LPS alone. Similar results were obtained with three other specimens.

These Examples indicate that ANP antagonizes the action of LPS by enhancing IL-10 production and reducing IL-12 production in dendritic cells, and it also polarizes the dendritic cells toward Th2-promoting phenotype and hence drives T cells to differentiate into Th2-type helper T cells, thereby inhibiting Th1-mediated immune reactions.

## Claims

1. A pharmaceutical composition for preventing or treating a Th1-mediated immune disease, which comprises as an active ingredient a substance capable of acting on the natriuretic peptide receptor guanylyl cyclase A to enhance the production of cyclic guanosine monophosphate.

2. The pharmaceutical composition according to claim 1, wherein the Th1-mediated immune disease is selected from a disease due to graft rejection following transplantation, graft-versus-host disease caused by bone marrow (hematopoietic stem cell) transplantation, and an autoimmune disease.

3. The pharmaceutical composition according to claim 2, wherein the autoimmune disease is selected from autoimmune hepatitis, chronic rheumatoid arthritis, insulin-dependent diabetes mellitus, ulcerative colitis, Crohn's disease, multiple sclerosis, autoimmune myocarditis, psoriasis, scleroderma, myasthenia gravis, multiple myositis/dermatomyositis, Hashimoto's disease, autoimmune hypocytosis (e.g., pure red cell aplasia, aplastic anemia), Sjogren's syndrome, vasculitis syndrome, and systemic lupus erythematosus.

4. The pharmaceutical composition according to claim 3, wherein the autoimmune disease is Crohn's disease or multiple sclerosis.

5. The pharmaceutical composition according to claim 1, wherein the substance capable of acting on the natriuretic peptide receptor guanylyl cyclase A to enhance the production of cyclic guanosine monophosphate is a natriuretic peptide.

6. The pharmaceutical composition according to claim 5, wherein the natriuretic peptide is atrial natriuretic peptide or brain natriuretic peptide.

7. The pharmaceutical composition according to claim 6, wherein the atrial natriuretic peptide is of human origin.

8. A method for treating a Th1-mediated immune disease, which comprises administering a substance capable of acting on the natriuretic peptide receptor guanylyl cyclase A to enhance the production of cyclic guanosine monophosphate.

9. The method according to claim 8, wherein the Th1-mediated immune disease is selected from a disease due to graft rejection following transplantation, graft-versus-host disease caused by bone marrow (hematopoietic stem cell) transplantation, and an autoimmune disease.

10. The method according to claim 9, wherein the autoimmune disease is selected from autoimmune hepatitis, chronic rheumatoid arthritis, insulin-dependent diabetes mellitus, ulcerative colitis, Crohn's disease, multiple sclerosis, autoimmune myocarditis, psoriasis, scleroderma, myasthenia gravis, multiple myositis/dermatomyositis, Hashimoto's disease, autoimmune hypocytosis (e.g., pure red cell aplasia, aplastic anemia), Sjogren's syndrome, vasculitis syndrome, and systemic lupus erythematosus.

11. The method according to claim 10, wherein the autoimmune disease is Crohn's disease or multiple sclerosis.

12. The method according to claim 8, wherein the substance capable of acting on the natriuretic peptide receptor guanylyl cyclase A to enhance the production of cyclic guanosine monophosphate is a natriuretic peptide.

13. The method according to claim 12, wherein the natriuretic peptide is atrial natriuretic peptide or brain natriuretic peptide.

14. The method according to claim 13, wherein the atrial natriuretic peptide is of human origin.

15. Use of a substance capable of acting on the natriuretic peptide receptor guanylyl cyclase A to enhance the production of cyclic guanosine monophosphate for the manufacture of a pharmaceutical composition for preventing or treating a Th1-mediated immune disease.

16. The use according to claim 15, wherein the Th1-mediated immune disease is selected from a disease due to graft rejection following transplantation, graft-versus-host disease caused by bone marrow (hematopoietic stem cell) transplantation, and an autoimmune disease.

17. The use according to claim 16, wherein the autoimmune disease is selected from autoimmune hepatitis, chronic rheumatoid arthritis, insulin-dependent diabetes mellitus, ulcerative colitis, Crohn's disease, multiple sclerosis, autoimmune myocarditis, psoriasis, scleroderma, myasthenia gravis, multiple myositis/dermatomyositis, Hashimoto's disease, autoimmune hypocytosis (e.g., pure red cell aplasia, aplastic anemia), Sjogren's syndrome, vasculitis syndrome, and systemic lupus erythematosus.

18. The use according to claim 17, wherein the autoimmune disease is Crohn's disease or multiple sclerosis.

19. The use according to claim 15, wherein the substance capable of acting on the natriuretic peptide receptor guanylyl cyclase A to enhance the production of cyclic guanosine monophosphate is a natriuretic peptide.

20. The use according to claim 19, wherein the natriuretic peptide is atrial natriuretic peptide or brain natriuretic peptide.

21. The use according to claim 20, wherein the atrial natriuretic peptide is of human origin.

22. A method for regulating the Th1/Th2 balance in the immune system, which comprises treating dendritic cells with a substance capable of acting on the natriuretic peptide receptor guanylyl cyclase A to enhance the production of cyclic guanosine monophosphate, and thereby polarizing T cells toward Th2-promoting phenotype.

23. The method according to claim 22, wherein the substance capable of acting on the natriuretic peptide receptor guanylyl cyclase A to enhance the production of cyclic guanosine monophosphate is a natriuretic peptide.

24. The method according to claim 23, wherein the natriuretic peptide is atrial natriuretic peptide or brain natriuretic peptide.

25. The method according to claim 24, wherein the atrial natriuretic peptide is of human origin.


[Sequence Listing]
<110> Daiichi Asubio Pharma Co., Ltd.
<120> PHARMACEUTICAL COMPOSITIONS FOR PREVENTING OR TREATING
      Th1-MEDIATED IMMUNE DISEASES
<130> 031317
<160> 16

<210> 1
<211> 28
<212> PRT
<213> Homo sapiens
<400> 1
Ser Leu Arg Arg Ser Ser Cys Phe Gly Gly Arg Met Asp Arg Ile Gly
                5                   10                  15
Ala Gln Ser Gly Leu Gly Cys Asn Ser Phe Arg Tyr
            20                  25

<210> 2
<211> 28
<212> PRT
<213> Rat
<400> 2
Ser Leu Arg Arg Ser Ser Cys Phe Gly Gly Arg Ile Asp Arg Ile Gly
                5                   10                  15
Ala Gln Ser Gly Leu Gly Cys Asn Ser Phe Arg Tyr
            20                  25

<210> 3
<211> 22
<212> PRT
<213> Homo sapiens
<400> 3
Cys Phe Gly Gly Arg Met Asp Arg Ile Gly Ala Gln Ser Gly Leu Gly
                5                   10                  15
Cys Asn Ser Phe Arg Tyr
            20

<210> 4
<211> 32
<212> PRT
<213> Homo sapiens
<400> 4
Ser Pro Lys Met Val Gln Gly Ser Gly Cys Phe Gly Arg Lys Met Asp
                5                   10                  15
Arg Ile Ser Ser Ser Ser Gly Leu Gly Cys Lys Val Leu Arg Arg His
            20                  25                  30

<210> 5
<211> 24
<212> PRT
<213> Frog
<400> 5
Ser Ser Asp Cys Phe Gly Ser Arg Ile Asp Arg Ile Gly Ala Gln Ser
                5                   10                  15
Gly Met Gly Cys Gly Arg Arg Phe
            20

<210> 6
<211> 32
<212> PRT
<213> Pig
<400> 6
Ser Pro Lys Thr Met Arg Asp Ser Gly Cys Phe Gly Arg Arg Leu Asp
                5                   10                  15
Arg Ile Gly Ser Leu Ser Gly Leu Gly Cys Asn Val Leu Arg Arg Tyr
            20                  25                  30

<210> 7
<211> 45
<212> PRT
<213> Rat
<400> 7
Ser Gln Asp Ser Ala Phe Arg Ile Gln Glu Arg Leu Arg Asn Ser Lys

                5                   10                  15
Met Ala His Ser Ser Ser Cys Phe Gly Gln Lys Ile Asp Arg Ile Gly
            20                  25                  30
Ala Val Ser Arg Leu Gly Cys Asp Gly Leu Arg Leu Phe
        35                  40                  45

<210> 8
<211> 29
<212> PRT
<213> Chick
<400> 8
Met Met Arg Asp Ser Gly Cys Phe Gly Arg Arg Ile Asp Arg Ile Gly
                5                   10                  15
Ser Leu Ser Gly Met Gly Cys Asn Gly Ser Arg Lys Asn
            20                  25

<210> 9
<211> 21
<212> DNA
<213> Artificial Sequence
<400> 9
gggaacctca agtcatccaa c

<210> 10
<211> 20
<212> DNA
<213> Artificial Sequence
<400> 10
atgaagggca aaggcaaggt

<210> 11
<211> 20
<212> DNA
<213> Artificial Sequence
<400> 11
tctagaaaat gacagcatca

<210> 12
<211> 20
<212> DNA
<213> Artificial Sequence
<400> 12
tgacaacttt gatgtctaca

<210> 13
<211> 24
<212> DNA
<213> Artificial Sequence
<400> 13
gaaggtatcg ccgggcaggt gtcc

<210> 14
<211> 24
<212> DNA
<213> Artificial Sequence
<400> 14
tcttcccgta attcccgatg tttt

<210> 15
<211> 21
<212> DNA
<213> Artificial Sequence
<400> 15
tcctgtggca tccacgaaac t

<210> 16
<211> 21

<212> DNA
<213> Artificial Sequence
<400> 16
gaagcatttg cggtggacga t